# EUROPÄISCHE PATENTANMELDUNG

(11) **EP 1 180 377 A1**
(43) Veröffentlichungstag der Anmeldung: **20.02.2002**
(21) Anmeldenummer: 00117646.0
(22) Anmeldetag: 16.08.2000
(51) Int. Cl.: A61M 5/315

(54) **Spritze mit Kolben**

(71) Anmelder: SCHERING AKTIENGESELLSCHAFT, 13342 Berlin (DE)
(72) Erfinder: Witowski, Norbert, Dr., 38302 Wolfenbüttel (DE)
(74) Vertreter: Prüfer, Lutz H., Dipl.-Phys.

(57) **Zusammenfassung**

Es wird eine Spritze mit einem Zylinder (1) und einem darin gleitenden Kolben (4) vorgesehen, der in seiner Stirnseite (5) eine Ausnehmung (13) aufweist und auf seiner Mantelfläche (6) mindestens zwei zylindrische Abschnitte (9, 10) aufweist, zwischen denen eine Rille (12) den Durchmesser des Kolbens (4) reduziert. Der in der Spritze aufgebrachte Druck wirkt über die äußere Flanke (14) der Ausnehmung und über einen äußeren Stirnflächenabschnitt (15) auf eine Dichtlippe (16), welche durch diesen Druck oder auch durch eine zuvor eingebrachte Vorspannung gegen die Zylinderinnenwand gedrückt wird und damit den Spritzeninhalt gegen die Umgebung abdichtet.

## Beschreibung

Die vorliegende Erfindung bezieht sich auf eine Spritze mit Kolben nach dem Oberbegriff des Anspruchs 1. Derartige Spritzen werden als vorgefüllte, sterilisierte Spritzen verwendet, die für den Einsatz von injizierbaren Diagnostika, insbesondere Kontrastmitteln, vorgesehen sind, welche zum Beispiel in Blutgefäße, Organe, Organteile, Höhlungen und andere Gefäße injiziert werden oder dort bildgebende Wirkung entfalten.

Aus der DE 196 44 622 A1 ist eine Spritze nach dem Oberbegriff des Anspruchs 1 bekannt. Durch die im Querschnitt spitz zulaufende Dichtlippe kann eine Formstabilität nur schlecht erreicht werden, so daß nur eine vergleichsweise geringe Vorspannung der Lippe in radialer Richtung gegen die Zylinderwand bewirkt werden kann. Eine Abdichtung der gefüllten Spritze im statischen Zustand mittels Dichtlippe wird dadurch erschwert.

Es stellt sich somit die Aufgabe, eine Spritze anzubieten, die im statischen Zustand der Lagerung ebenso wie bei der dynamischen Belastung mit Erzeugung eines Drucks in der Spritze (wie im Falle der Benutzung oder des Autoklavierens) eine ausreichende Abdichtung des Inhalts der Spritze gegenüber dem umliegenden Bereich gewährleistet.

Die Aufgabe wird gelöst durch eine Spritze nach Anspruch 1.

Die Spritze weist insbesondere einen Zylinder mit innenliegendem Kolben auf, der den Zylinder in proximaler Richtung verschließt. Die Spritze weist einen Kolben auf, der mit seiner speziell geformten Dichtlippe im Falle eines dynamischen Einsatzes vorteilhafterweise eine stärkere Dichtung als im statischen Einsatz gegenüber der Innenwand der Spritze aufweist. Die Spritze zeigt weiterhin den Vorteil, daß der Kolben mit einer einfach geformten Stirnfläche gefertigt werden kann, welche auch das Einbringen der Ausnehmung in die Stirnfläche mit geringem technischem Aufwand möglich macht. Die zylindrischen Abschnitte der Mantelfläche verbessern die Führung des Kolbens im Zylinder und verhindern ein Verkanten.

Bevorzugte Ausgestaltungen der Erfindung ergeben sich aus den Unteransprüchen.

Die Ausführung nach Anspruch 3 zeigt den Vorteil, daß in der ausgedrückten Spritze zwischen der Stirnfläche des Kolbens und der gleich geformten Innenfläche des Verschlusses ein Hohlraum weitestgehend vermieden wird. Dadurch wird ein verbleibendes Restvolumen in der Spritze minimiert.

Die Ausführung nach Anspruch 4 zeigt den Vorteil einer einfachen Formgebung bei der Herstellung des Kolbens.

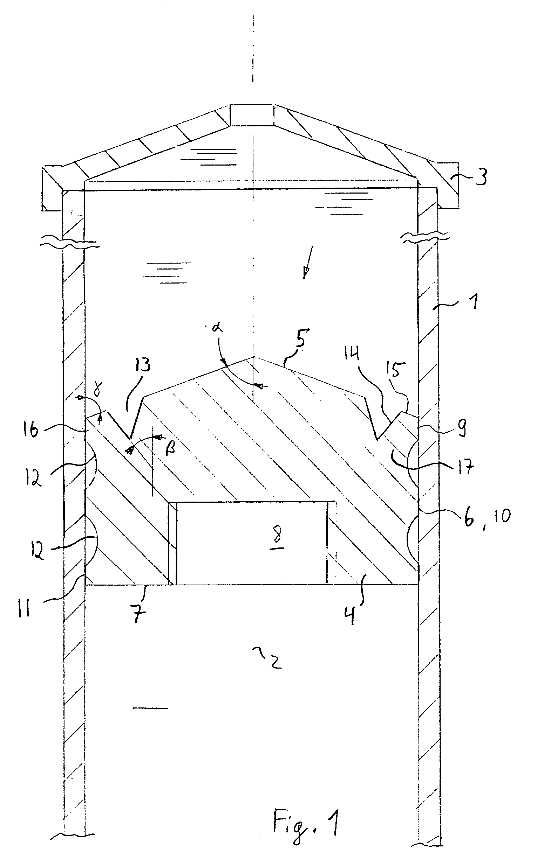

Weitere Zweckmäßigkeiten und Vorteile der Erfindung ergeben sich anhand der Beschreibung eines Ausführungsbeispiels nach Fig. 1. Fig. 1 zeigt einen Ausschnitt einer Querschnittsansicht einer ersten Ausführungsform der Spritze.

Wie in Fig. 1 dargestellt ist, ist ein Zylinder 1 mit einer Achse 2 vorgesehen. Der Zylinder besteht bevorzugt aus Glas oder Kunststoff. Der Zylinder 1 ist an seinem distalen Ende durch einen Verschluß 3 in bekannter Weise verschlossen. Ein Kolben 4 ist in dem Zylinder 1 axial verschieblich angeordnet. Die Achse 2 fällt mit der Achse des Kolbens 4 zusammen.

Der Kolben 4 weist eine Stirnfläche 5, eine Mantelfläche 6 und eine Rückfläche 7 auf. In der Rückfläche 7 ist zur Aufnahme einer Kolbenstange eine Aufnahmeöffnung 8 zentrisch angeordnet und mit einem Gewinde versehen. Das bevorzugten Material des Kolbens 4 sind Elastomere. Bei Gummi handelt es sich um ein Elastomer, welches ein vulkanisierter Kautschuk ist. Kautschuk bezeichnet unvernetzte, aber vernetzbare (vulkanisierbare) Polymere mit gummielastischen Eigenschaften bei Raumtemperatur.

Die Mantelfläche 6 weist angrenzend an die Stirnfläche 5 einen ersten zylinderförmigen Abschnitt 9 auf. Ein zweiter zylinderförmigen Abschnitt 10 und ein dritter zylinderförmigen Abschnitt 11 sind in axial gesehenen Abständen von dem ersten zylinderförmigen Abschnitt 9 auf der Mantelfläche 6 angeordnet. Zwischen dem ersten zylinderförmigen Abschnitt 9 und dem zweiten zylinderförmigen Abschnitt 10 sowie zwischen dem zweiten zylinderförmigen Abschnitt 10 und dem dritten zylinderförmigen Abschnitt 11 weist die Mantelfläche 6 jeweils eine umlaufende ringförmige Rille 12 auf. Der Durchmesser des Kolbens 4 ist im Bereich der Rille 12 daher geringer als in dem ersten, zweiten oder dritten zylinderförmigem Abschnitt (9, 10, 11). Die Rille 12 ist im Querschnitt konkav ausgebildet.

Die Stirnfläche 5 ist um die Achse 2 konisch und in distaler Richtung spitz zulaufend ausgebildet. Zwischen der Stirnfläche 5 und der Achse 2 wird im Querschnitt ein Winkel α eingeschlossen. Der Winkel α wird bevorzugt größer als 30° gewählt. Noch bevorzugter liegt der Winkel α zwischen 45° und 90°. In der Fig. 1 ist der Winkel α gleich 70°.

In der Stirnfläche 5 des Kolbens 4 ist in einem Abstand von der Mantelfläche 6 eine konzentrisch zur Achse 2 verlaufende ringförmige Ausnehmung 13 mit einer Flanke 14 vorgesehen. Die Ausnehmung 13 ist V-förmig ausgebildet. Die Tiefe der Ausnehmung 13 in axialer Richtung ist im wesentlichen gleich ihrem radialen Abstand von der Mantelfläche 6. Die Flanke 14 der Ausnehmung 11 bildet mit ihrer gedachten Verlängerung durch die Achse 2 einen Winkel β. Der Winkel β ist bevorzugt kleiner als 90°. Noch bevorzugter liegt der Winkel β zwischen 20° und 60°. In der Fig. 1 ist der Winkel β gleich 40°.

Durch die Ausnehmung 13 wird ein äußerer Stirnflächenabschnitt 15 gebildet, welcher an den ersten zylindrischen Abschnitt 9 angrenzt. Zwischen dem äußeren Stirnflächenabschnitt 15 und der Achse 2 im Querschnitt wird ein Winkel γ eingeschlossen. Der Winkel γ wird bevorzugt gleich dem Winkel α zwischen der Stirnfläche 5 und der Achse 2 im Querschnitt gewählt. Der Winkel γ ist bevorzugt größer als 30°. Noch bevorzugter liegt der Winkel γ zwischen 45° und 90°. In der Fig. 1 ist der Winkel γ gleich 70°.

Die Flanke 14 bildet zusammen mit dem äußeren Stirnflächenabschnitt 15, dem ersten zylindrischen Abschnitt 9 und der an den ersten zylindrischen Abschnitt 9 anschließenden Rille 12 eine Dichtlippe 16. Die Dichtlippe 16 liegt über den ersten zylinderförmigen Abschnitt 9 an der Innenwand des Zylinders 1 an. Die Dichtlippe 16 ist über ihre Basis 17 mit dem Kolben 4 verbunden.

Die Spritze wird üblicherweise nach der Befüllung über längere Zeit gelagert. Folglich muß der Kolben 4 außer unter dynamischer Last auch im statischen Zustand den eingeschlossenen Spritzeninhalt sicher gegenüber dem proximalen Ende des Zylinders 1 abdichten. Der Kolben 4 erfüllt diese Anforderung durch die zuvor beschriebenen Eigenschaften auf dreifache Weise:

Durch die Breite der Basis 17 und die wenigstens im Bereich der Basis 17 gegebene Verformbarkeit des Kolbens 4 kann die an der Basis 17 mit dem Kolben 4 verbundene Dichtlippe 16 vor dem Einsetzen in den Zylinder 1 in radialer Richtung elastisch so vorgespannt werden, daß sie im eingesetzten Zustand mit der eingebrachten Vorspannkraft statisch gegen die Zylinderinnenwand drückt.

Durch die gegenüber der Achse 2 geneigte Flanke 14 der Ausnehmung 13 resultiert aus dem Druck im Spritzeninneren im Zylinder 1 eine statische und eine dynamische Kraftkomponente in radialer Richtung auf die Dichtlippe 16, welche diese zusätzlich gegen die Zylinderinnenwand drückt.

Durch die zylindrische Außenform des ersten zylindrischen Abschnitts 9 wird sichergestellt, daß die Dichtlippe 16 nicht nur über einen Linienkontakt an der Zylinderinnenwand anliegt sondern über eine zylindrische Fläche. Mögliche Unebenheiten im Kontaktbereich (und damit verbundene Undichtigkeiten) mit der Zylinderinnenwand können durch die in axialer Richtung ausgedehnte Kontaktzone überbrückt und ausgeglichen werden.

Die Form der Dichtlippe 16 so zu wählen, daß die Kraft, die durch die Reibung der Dichtlippe 16 gegenüber der Zylinderinnenwand in proximale Richtung auftritt und diejenige axial gerichtete Kraft, welche durch den Druck im Fluid auf den äußeren Stirnflächenabschnitt 15 in axialer Richtung ein Kippmoment um die Basis 16 bewirkt, kleiner sind als die Kräfte in der Basis 17, welche die Dichtlippe in ihrer Form stabilisieren. Dadurch wird ein Umschlagen der Dichtlippe 16 in proximaler Richtung verhindert.

In einer weiteren Ausführungsform kann die Ausnehmung 13 mit unterschiedlichen Querschnittsformen ausgebildet werden. So kann die Flanke 14 in gebogener oder gewellter Form ausgebildet sein.

In einer weiteren Ausführungsform erstreckt sich die Ausnehmung 13 mit ihrer Tiefe in radialer Richtung von der Dichtlippe 16 bis zur Achse 2, so daß die Ausnehmung 13 die Form einer Scheibe annimmt.

In einer weiteren Ausführungsform ist die Tiefe der Ausnehmung 13 in axialer Richtung im wesentlichen gleich ihrem radialen Abstand von der Mantelseite 6. Abhängig von der Form der Ausnehmung 13 und dem Kolbenmaterial können andere Verhältnisse von Abstand zu Tiefe gewählt werden, etwa um die Steifigkeit des Kolbens 4 im Bereich der Basis 17 mit der daran angrenzenden Dichtlippe 16 zu optimieren.

In einer weiteren Ausführungsform ist die Rille 12 im Querschnitt nicht konkav, sondern stufenförmig mit einem oder mehreren Absätzen gebildet. Ebenso kann die Rille 12 durch linear verlaufende Durchmesserreduzierungen oder durch eine Kombination der vorgenannten Formen gebildet werden.

In einer weiteren Ausführungsform weist der Kolben einen starren Kern auf. Dadurch werden die aufgebrachten Schubkräfte gleichmäßig in den gesamten Kolben eingebracht.

## Patentansprüche

1. Spritze mit einem Zylinder (1), einer Symmetrieachse (2), einem ausgangsseitigen Verschluß (3) an einem Ende und einem eingangsseitigen Kolben (4) mit einer Stirnfläche (5), einem Mantel (6) und einer der Stirnfläche gegenüberliegenden Rückfläche (7),
wobei der Mantel (6) angrenzend an die Stirnfläche (5) einen ersten Abschnitt (9) eines ersten Durchmessers und in einem Abstand davon wenigstens einen zweiten Abschnitt (10) des ersten Durchmessers und dazwischen einen Bereich kleineren Durchmessers aufweist und in der Stirnfläche (5) in einem vorgegebenen Abstand von dem Rand eine Ausnehmung (13) vorgesehen ist,
**dadurch gekennzeichnet, daß** der erste Abschnitt (9) als zylinderförmiger Abschnitt ausgebildet ist und die Ausnehmung (13) auf der Mantelseite eine sich von der Stirnfläche (5) aus zur Symmetrieachse (2) hin geneigte Seitenfläche (14) aufweist.

2. Spritze mit einem Zylinder nach Anspruch 1, **dadurch gekennzeichnet, daß** die Ausnehmung (13) ringförmig ist.

3. Spritze mit einem Zylinder nach Anspruch 2, **dadurch gekennzeichnet, daß** die Ausnehmung (13) einen V-förmigen Querschnitt aufweist.

4. Spritze mit einem Zylinder nach einem der Ansprüche 1 bis 3, **dadurch gekennzeichnet, daß** die Stirnfläche (5) zum ausgangsseitigen Verschluß (3) hin konisch ausgebildet ist.

5. Spritze mit einem Zylinder nach einem der Ansprüche 1 bis 4, **dadurch gekennzeichnet, daß** der Bereich (12) zwischen dem ersten (9) und zweiten Abschnitt (10) konkav ausgebildet ist.

6. Spritze mit einem Zylinder nach einem der Ansprüche 1 bis 5, **dadurch gekennzeichnet, daß** angrenzend an die Rückfläche (7) ein zylinderförmiger Abschnitt des ersten Durchmessers vorgesehen ist.

7. Spritze mit einem Zylinder nach einem der Ansprüche 1 bis 6, **dadurch gekennzeichnet, daß** der radiale Abstand der Ausnehmung (13) von dem äußeren Rand der Stirnfläche (5) etwa gleich ihrer Tiefe ist.
